# EUROPÄISCHE PATENTSCHRIFT

(11) **EP 2 602 247 B1**
(45) Veröffentlichungstag und Bekanntmachung des Hinweises auf die Patenterteilung: **14.05.2014**
(21) Anmeldenummer: 11192425.4
(22) Anmeldetag: 07.12.2011
(51) Int. Cl.: C07D 207/267

(54) **Verfahren zur Reinigung von N-alkyl-substituierten Pyrrolidonen durch Hydrierung**
Method for purifying N-alkyl substituted pyrrolidones through hydration
Procédé de nettoyage de pyrrolidones N-alkyl-substitués par hydrogénisation

(43) Veröffentlichungstag der Anmeldung: 12.06.2013
(73) Patentinhaber: BASF SE, 67056 Ludwigshafen (DE)
(72) Erfinder: Pinkos, Rolf, 67098 Bad Dürkheim (DE); Vogler, Thomas, 67071 Ludwigshafen (DE); Ott, Karl, 68723 Plankstadt (DE)

(56) Entgegenhaltungen:
- EP-A1- 0 609 752
- JP-A- 11 071 346
- JP-A- 2007 099 690

## Beschreibung

Die Erfindung betrifft ein Verfahren zur Reinigung von N-Alkyl-substituierten Pyrrolidonen, die Verunreinigungen mit höherem Oxidationsgrad aufweisen, durch Hydrierung derselben und Destillation.

N-Alkyl-substituierte Pyrrolidone werden technisch durch die Umsetzung von Alkylaminen mit gamma-Butyrolacton hergestellt. So erhält man beispielsweise durch Umsetzung von Methylamin mit gamma-Butyrolacton N-Methylpyrrolidon (NMP), das technisch vielfältig als Lösungsmittel eingesetzt wird, wie von K. Weissermel, H.-J. Arpe in Industrielle Organische Chemie, 5.

Auflage, Wiley-VCH, Seite 115 beschrieben. Die Reingewinnung des NMP erfolgt durch destillative Abtrennung von ggf. überschüssigem Methylamin sowie Reaktionswasser. Die erzielbaren Reinheiten sind sehr hoch und liegen deutlich über 99,5 %, gewöhnlich über 99,8 %. Andere Alkylamine, wie z.B. N-Ethylpyrrolidon, werden analog hergestellt.

Beim Einsatz von N-Alkylpyrrolidonen, z.B. als Lösemittel, werden diese in ihrer Zusammensetzung geändert, was einer direkten Wiederverwendung entgegensteht, obwohl diese, aus umwelttechnischen und ökonomischen Gründen, sehr wünschenswert wäre.

Eine Anwendung für N-Alkyl-substituierte Pyrrolidone und insbesondere NMP ist die Herstellung von Lithiumionen-Batterien als Lösungsmittel für die Beschichtungen der Anoden- und Kathodenträger. Hierfür sind besondere Anforderungen an das Lösemittel gestellt, insbesondere sollte die eingesetzte Qualität nicht signifikant schwanken und keine neuen, zuvor nicht auf ihren Prozesseinfluss getestete Nebenkomponenten, enthalten. So sollen die Metallionengehalte unter 5 ppb, Wassergehalte unter 300 ppm , die Gesamtreinheit (inklusive von 1,3- und 1,4-Dimethylpyrrolidion) über 99,8 % (sogenannte "electronic grade quality") liegen. Nach der ersten Verwendung von N-Alkyl-substituierten Pyrrolidonen kann es neben der Erhöhung des Wassergehalts, zur Verunreinigung z.B. durch chemische Reaktion kommen, was eine wünschenswerte direkte Wiederverwendung des N-Alkyl-substituierten Pyrrolidons und insbesondere die Verwendung in der Herstellung von Lithiumionen-Batterien verhindert.

In WO 2011/030728 A1 wird eine Destillation von NMP beschrieben. Wie das Vergleichsbeispiel 1 dieser Anmeldung zeigt, in dem NMP, Verunreinigungen enthält, die einen höheren Oxidationsgrad als NMP selbst aufweisen, kann eine Destillation wie sie in WO 2011/030728 A1 beschrieben ist, nicht dazu eingesetzt werden, um die nötigen Reinheitsgrade des ursprünglich eingesetzten NMPs wieder zu erreichen. Reinheitsgrade wie das frisch eingesetzte N-Alkylpyrrolidon sind daher durch reine Destillation nicht zu erreichen und somit für das Recyceln von N-Alkyl-substituierten Pyrrolidonen, die in der Herstellung von Lithiumionen-Batterien eingesetzt werden, ungeeignet.

Für die Reinigung von N M P schlägt die Firma AMCEC auf der Internetseite http://www.amcec.com/nmp%20recovery.html ("AMCEC NMP Recovery System") vor, dass Verunreinigungen, per Filtration über Aktivkohle oder Zeolithe entfernt werden können. Gemäß dem in dieser Anmeldung durchgeführten Vergleichsbeispiel 2 sind jedoch N-Alkylpyrrolidone wie NMP, das Verunreinigungen mit höherem Oxidationsgrad als NMP enthält, nicht auf diese Art zu reinigen. Daher ist auch diese Reinigung per Filtration nicht geeignet für den Einsatz des N-Alkyl-substituierten Pyrrolidons als Lösungsmittel in der Batterieherstellung mit electronic grade quality.

Die Aufgabe der vorliegenden Erfindung ist es daher ein Verfahren bereit zu stellen, dass es ermöglicht, auf einfachem Wege, effizient und kostengünstig mit jedoch hohen Ausbeuten, N-Alkyl-substituierte Pyrrolidone in hohen Reinheitsgraden, die dem electronic grade entsprechen, wiederzugewinnen.

Diese Aufgabe wird gelöst durch ein Verfahren zur Reinigung von N-Alkyl-substituierten Pyrrolidonen, die eine oder mehrere der Verunreinigungen der Formel I bis VII enthalten, 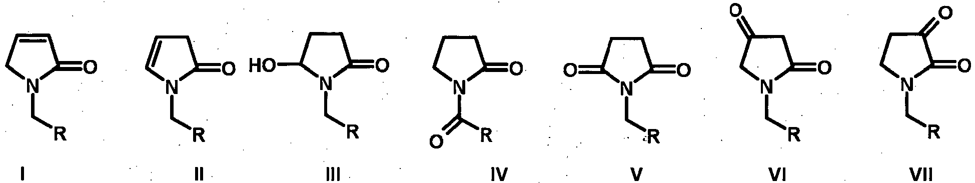 mit R ausgewählt aus der Gruppe von Wasserstoff, linearen oder verzweigten C₁-C₂₀-Alkylgruppen,
umfassend folgende Schritte
I) Bereitstellung einer Mischung enthaltend mindestens ein N-Alkyl-substituiertes Pyrrolidon, mindestens eine Verbindung der Formel I bis VII in Mengen von 1 bis 20000 ppm
II) Hydrierung der Mischung aus Schritt I)
III) Destillation der erhaltenen Mischung aus Schritt II).

In den Verunreinigungen der Formel I bis VII steht der Rest R für Wasserstoff, lineare oder verzweigte C₁-C₂₀-Alkylreste. Bevorzugt ist R Wasserstoff oder ein linearer oder verzweigter C₁-C₁₀ Alkylrest. Besonders bevorzugt ist R ausgewählt aus der Gruppe von Wasserstoff, Methyl, Ethyl, n-Propyl und n-Butyl. Ganz besonders bevorzugt ist R ausgewählt aus der Gruppe von Wasserstoff und Methyl. Insbesondere ganz besonders bevorzugt ist R Wasserstoff.

Die Menge dieser Verunreinigungen der Formel I bis VII im N-Alkyl-substituierten Pyrrolidon liegt im Allgemeinen einzeln oder als Mischung einzelner oder aller im Bereich von 1 bis 20000 ppm, bevorzugt zwischen 2 und 15000 ppm, besonders bevorzugt zwischen 5 und 10000 ppm, bezogen auf die Menge der zu reinigenden Mischung. Im zu reinigenden N-Alkyl-substituierten Pyrrolidon können zu den Verunreinigungen der Formel I bis VII auch deren alkyl-substituierte Derivate oder Oxo-verbrückte Verunreinigungen einzeln oder als Mischung einzelner oder aller vorliegen. In den Verunreinigungen steht der Rest R1 für lineare oder verzweigte C₁-C₂₀-Alkylreste. Bevorzugt ist R1 ein linearer oder verzweigter C₁ - C₁₀ Alkylrest. Besonders bevorzugt ist R1 ausgewählt aus der Gruppe Methyl oder Ethyl.

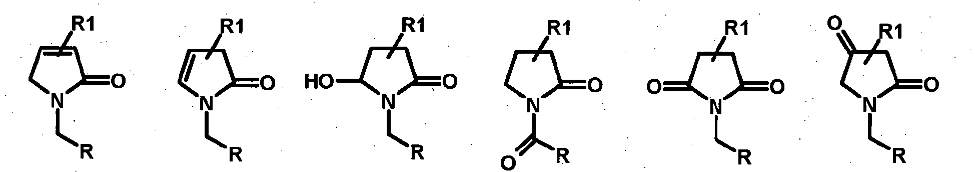

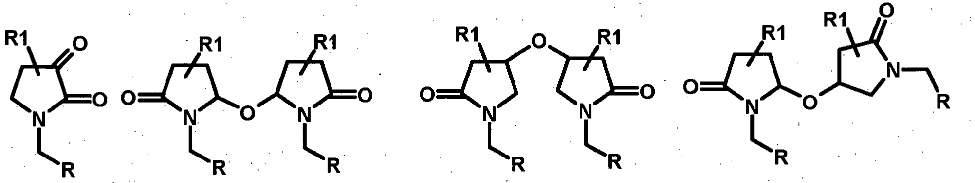

Der Wassergehalt des zu reinigenden N-Alkyl-substituierten Pyrrolidon liegt im Allgemeinen zwischen 0,05 % bis 90 %, bevorzugt zwischen 0,1 % bis 50 % und besonders bevorzugt zwischen 0,2 % - 10 %.

Die Hydrierung gemäß Schritt II) des erfindungsgemäßen Verfahrens kann mit Hilfe von Wasserstoff und hydrieraktiven Katalysatoren oder durch den Einsatz von komplexen Hydriden erfolgen.

Bei der Hydrierung oder Reduktion mittels komplexer Hydride, sind diejenigen bevorzugt ausgewählt, die in Advanced Organic Chemistry, J. March, 3.Auflage J. Wiley & Sons, 1985, Seiten 809 - 814 angegeben sind. Besonders bevorzugt sind Natriumborhydrid und Lithiumaluminiumhydrid.

Die komplexen Hydride werden beispielsweise in Mengen zwischen 5 und 50000 ppm, bevorzugt zwischen 50 bis 25000 ppm, besonders bevorzugt zwischen 100 und 10000 ppm bezogen auf die Menge der zu reinigenden Mischung zugesetzt. Die Menge an komplexen Hydriden richtet sich nach der Menge an Verunreinigung, das komplexe Hydrid ist mindestens stöchiometrisch, bevorzugt aber überstöchiometrisch zur Menge an Verunreinigung einzusetzen.

Die komplexen Hydride können in Substanz oder in Lösung oder als Suspension eingebracht werden. Als Lösemittel sind bevorzugt Wasser, sofern es nicht mit dem Hydrid reagiert, das herzustellende N-Alkyl-substituierte Pyrrolidon, Ether wie z.B. Tetrahydrofuran und Diethylether. Besonders bevorzugt ist das herzustellende N-Alkyl-substituierte Pyrrolidon. Bei der Verwendung von NaBH₄ wird insbesondere bevorzugt Wasser als Lösungsmittel verwendet.

Die Zugabe des komplexen Hydrids, das zum verunreinigten N-Alkyl-substituierten Pyrollidon, insbesondere zum verunreinigen NMP, hinzugefügt wird, kann bei Temperaturen zwischen 10 und 350 °C erfolgen. Bevorzugt ist das erfindungsgemäße Verfahren, wenn die Temperatur und Verweilzeit des komplexen Hydrids auf einander abgestimmt ist. Generell gilt, je höher die Temperatur, desto geringer ist die notwendige Verweilzeit, die benötigt wird, um den gewünschten Reinigungseffekt zu erhalten. So liegen die Verweilzeiten beispielsweise von 0,1 bis 10 Stunden bei Temperaturen zwischen 50 und 250 °C, wobei während des erfindungsgemäßen Verfahrens die Temperatur nicht konstant gehalten werden muss. Die notwendige Verweilzeit für den Prozess setzt sich zusammen aus der Verweilzeit, in der das verunreinigte N-Alkyl-substituierte Pyrrolidon zusammen mit dem komplexen Hydrid umgesetzt wird und der Verweilzeit, die während der Destillation eingestellt wird. Ist die Verweilzeit während der Destillation bereits ausreichend, so kann ein separater Verweilzeitschritt entfallen.

Die Destillation kann diskontinuierlich oder kontinuierlich erfolgen. Dabei können für den Fachmann alle bekannten Destillationskolonnen eingesetzt werden.

Beispielsweise kann man, beim Einsatz eines absatzweisen Prozesses, wie folgt vorgehen: Man legt in einem ersten Schritt das verunreinigte N-Alkyl-substituierte Pyrrolidon, insbesondere NMP, zusammen mit dem komplexen Hydrid in einem Rührkessel, bevorzugt in einer Inertgasatmosphäre, vor, vermischt die Stoffe z.B. durch Rühren oder Umpumpen und heizt, ggf. unter Druck, auf die gewünschte Temperatur auf und lässt das Gemisch reagieren. Anschließend pumpt man im zweiten Schritt den Inhalt in eine oder mehrere Kolonnen und trennt (absatzweise oder kontinuierlich) Leichtsieder wie z.B. Wasser und Hochsieder, die ggf. das im Überschuss zugegebene komplexe Hydrid und gebildete Hochsieder enthalten, vom N-Alkyl-substituierten Pyrrolidon ab.

Der zuvor beschriebene erste Schritt, kann auch in einer Kolonne ablaufen, bevor destilliert wird.

Der zuvor beschriebene erste Schritt kann ebenso kontinuierlich durchgeführt werden. Dabei kann wiederum ein der Destillation vorgeschalteter separater Behälter oder eine Destillationskolonne verwendet werden.

Eine bevorzugte Ausführungsform des Verfahrens ist, das verunreinigte N-Alkyl-substituierte Pyrrolidon und insbesondere das verunreinigte NMP, sowie ein komplexes Hydrid zu einem Reaktionsaustrag hinzuzugeben der durch Umsetzung von gamma-Butyrolacton mit Methylamin zu NMP entstanden ist.

Dieses Gemisch enthält im Allgemeinen N-Alkyl-substituiertes Pyrrolidon, Wasser und das entsprechende Amin. Diese Verfahrensvariante wird bevorzugt kontinuierlich ausgeübt. Dabei wird in einer ersten Kolonne ein Gemisch von Amin, insbesondere Methylamin für die Reinigung von NMP, und Wasser über Kopf abgetrennt (Kolonnendruck 300 - 5000 mbar, Sumpftemperaturen 100 - 250 °C für die Reinigung von verunreinigtem N M P), das Sumpfprodukt gelangt in eine zweite Kolonne (Kolonnendruck 20 - 500 mbar, Sumpftemperaturen 120 bis 230 °C, für die Reinigung von verunreinigtem NMP), bei der Reste an Wasser und Amin, insbesondere Methylamin für die Reinigung von N M P, über Kopf abgetrennt werden und das Sumpfprodukt in eine dritte Kolonne (Kolonnendruck 20 - 300 mbar, Sumpftemperaturen 120 bis 230 °C, für die Reinigung von verunreinigtem NMP) gelangt, bei dem reines N-Alkyl-substituiertes Pyrrolidon, insbesondere NMP, über Kopf oder über einen Seitenabzug abgetrennt wird, während über Sumpf Hochsieder ausgeschleust werden.

Es gibt bei dieser Destillationssequenz auch Varianten, wie z.B. dass in der zweiten Kolonne über Kopf Wasser und Amin, insbesondere Methylamin für die Reinigung von NMP, ausgeschleust werden, über Seitenabzug N-Alkyl-substituiertes Pyrrolidon, insbesondere NMP, und über Sumpf Hochsieder. Das N-Alkyl-substituierte Pyrrolidon, insbesondere NMP, kann je nach Reinheitsanforderungen dann in einer dritten Kolonne, wie beschrieben, weiter gereinigt werden. Ist die zweite Kolonne als Trennwandkolonne ausgeführt, so kann das N-Alkyl-substituierte Pyrrolidon, insbesondere NMP, als Seitenabzug bereits sehr rein gewonnen werden und eine dritte Kolonne kann entfallen.

Die verwendeten Apparate sind aus gängigen Edelstählen gefertigt, ebenso alle Rohrleitung und Kolonneneinbauten wie Packungen usw. Um N-Alkyl-substituierte Pyrrolidone, insbesondere NMP in "Electronic-grade"-Qualität zu erhalten, empfiehlt es sich, auch die verwendeten Tanks in Edelstahl auszuführen und mit Schutzgas wie z.B. Stickstoff zu überlagern. Ggf. wird das N-Alkyl-substituierte Pyrrolidon, insbesondere NMP, zur Entfernung von Metallionen und/oder freien Aminspuren zusätzlich noch über lonentauscher geleitet.

Eine weitere Verfahrensvariante ist die Hydrierung mit Wasserstoff. Die Hydrierung mit Wasserstoff erfolgt in Gegenwart von hydrieraktiven Katalysatoren. Diese Katalysatoren enthalten Elemente des Periodensystems ausgewählt aus der Gruppe von Cobalt, Rhodium, Ruthenium, Iridium, Nickel, Palladium, Platin, Kupfer, Silber, Gold oder Rhenium. Besonders bevorzugt sind die Elemente ausgewählt aus der Gruppe von Nickel, Ruthenium, Palladium, Platin und Kupfer. Die Katalysatoren enthalten die Elemente in Form ihrer Metalle und/oder in Form unlöslicher Verbindungen z.B. Oxide oder sind homogen lösliche Metallkomplexe.

Die Hydrierkatalysatoren können als homogene lösliche Katalysatoren oder als Heterogenkatalysatoren eingesetzt werden. Wird ein homogen löslicher Katalysator eingesetzt so enthält dieser bevorzugt Ruthenium. Besonders bevorzugt ist der Einsatz von heterogenen Katalysatoren.

Die möglichen Elemente, die in den heterogenen Katalysatoren eingesetzt werden können, können einzeln und/oder als Mischung zum Einsatz kommen. Sie können beispielsweise als Voll-, Tränk- oder Fällkatalysatoren eingesetzt werden, mit oder ohne Dotierstoffe. Beispiele für Träger oder Gerüstmaterialen der Katalysatoren sind Kohle wie Aktivkohle oder Graphit, Aluminium-, Silizium-, Titan-, Cer- oder Zirkonoxide. Der Anteil an Hydrierelement an den Katalysatoren kann zwischen 0,001 bis 90 Gew.-% liegen. Bei Tränkkatalysatoren liegen die Gehalte bevorzugt bei 0,001 bis 20 Gew.-%, besonders bevorzugt bei 0,01 bis 10 Gew.-%, insbesondere bevorzugt bei 0,1 bis 5 Gew.-%.
Ganz besonders bevorzugte heterogene Katalysatoren sind Pd, Pt, Ru, Cu auf Aktivkohle.

Bevorzugt ist die katalytische Hydrierung bzgl. Druck und Temperatur unter möglichst milden Bedingungen durchzuführen, damit das N-Alkyl-substituierte Pyrrolidon, insbesondere das NMP, nicht selbst z.B. zum Pyrrolidin hydriert wird, was einen Verlust darstellen würde. Deshalb sind Drücke bis zu 320 bar zwar möglich, jedoch bis 100 bar bevorzugt und besonders bevorzugt bis 50 bar. Die untere Grenze liegt bevorzugt bei Normaldruck. Die Temperaturen liegen im Allgemeinen bei 20 bis 250 °C, bevorzugt bei 20 bis 200 °C, besonders bevorzugt bei 20 bis 150°C.

Die heterogen katalysierte Hydrierung kann im Festbett oder in Suspension durchgeführt werden. Bevorzugt ist Festbett in Riesel- oder Sumpffahrweise mit oder bevorzugt ohne Flüssigumlauf. Der Feed wird zusammen mit Wasserstoff über das Katalysatorbett geleitet. Der Reaktionsaustrag wird zumindest auf das Druckniveau entspannt, das in der nachfolgenden Destillation eingestellt ist.

Die Bedingungen für den erfindungsgemäßen Destillationsschritt III) sind die gleichen wie für die Hydrierung mittels eingesetzter komplexer Hydride.

### Beispiele

Zur Verdeutlichung der Erfindung sollen die nachfolgenden Beispiele dienen. Die in den Beispielen angegebenen Gehalte an Verunreinigungen sind durch Gaschromatographie (GC-Gerät HP6890, FID-Dektor, Stickstoff Trägergas mit 1,0 mL/min (const. flow); Split Ratio 1:50; Säule RTX-1, 30 m, 0,32 mm, 1,0 µm Film; Temperaturprogramm Start bei 80 °C, dann 5 °C/min bis 140 °C, dann 5 °C/min bis 200 °C und 10 min isotherm, dann 10 °C/min bis 340 °C und 8 min isotherm) bestimmt. Alle Versuche wurden unter einer Stickstoffatmosphäre durchgeführt. Wasserwerte wurden per Karl-Fischer-Titration bestimmt. Eingesetzt wurden N M P sowie die entsprechenden Verunreinigungen wie in den Beispielen angegeben.

### Vergleichsbeispiel 1

Destillation, wie sie in WO-A1 2011/030728 beschrieben wird.

Ein verunreinigtes NMP, mit 3 % Wasser und 500 ppm Verunreinigungen ausgewählt aus der Gruppe von Dehydro-NMP (Verunreinigung der Formel I), Oxo-NMP (Verunreinigung der Formel VI) wurde fraktioniert in einer 110 cm langen Füllkörperkolonne (Sulzer-Pack) mit Rücklauf teiler bei Sumpftemperaturen zwischen 130 bis 140 °C und Drücken zwischen zu Beginn 1000 mbar (Wasserabtrennung) und 90 mbar Kopfdruck bei einem Rücklauf- zu Abnahme-Verhältnis von 50 zu 1 destilliert. Es wurden ausgehend von 2 kg Vorlage, insgesamt 16 Destillatfraktionen genommen. In den ersten beiden Fraktionen, die bei Kopftemperaturen von 55 - 118 °C erhalten wurden, fand sich überwiegend Wasser (> 98 %). Bei 100 mbar und einer Kopftemperatur von 122 - 126 °C wurde eine Fraktion mit 0,7 % Wasser und NMP mit über 99 % erhalten. Die nachfolgenden Fraktionen 4 bis 13 (zusammen ca. 1,5 kg) enthielten Wasser unter 1000 ppm, NMP in Reinheiten über 99,8 % allerdings noch die bereits in der Vorlage vorhandenen Verunreinigungen. Ab Fraktion 14 (zusammen ca. 300 g) war das NMP frei von den unerwünschten Verunreinigungen. Der Versuch zeigt, dass alleine durch Destillation, nur ein unwirtschaftlich kleiner Teil an NMP (ca. 15 %) als NMP mit gewünschter Reinheit wiedergewonnen werden kann.

### Vergleichsbeispiel 2

Das Edukt wie in Vergleichsbeispiel 1 wurde über 500 mL Aktivkohle filtriert und anschließend wie in Vergleichsbeispiel 1 destilliert. Bereits nach der Filtration zeigte die GC-Analyse, dass kein Abreicherungseffekt oder organischen Verunreinigungen eingetreten war und auch das Destillationsergebnis war analog Vergleichsbeispiel 1.

### Vergleichsbeispiel 3

Vergleichsbeispiel 2 wurde wiederholt, anstelle von Aktivkohle wurde ein Zeolith verwendet. Es ergab sich das gleiche Ergebnis wie in Beispiel 2.

### Erfindungsgemäße Beispiele

### Beispiel 1

Das Einsatzgemisch aus Vergleichsbeispiel 1 wurde mit 1000 ppm NaBH₄ versetzt und 0,5 h auf 105 °C erhitzt. Lt. gaschromatographischer Analytik, waren die unerwünschten Verunreinigungen nicht mehr nachweisbar. Eine anschließende Destillation wie in Vergleichsbeispiel 1 ergaben ca. 95 % NMP in der gewünschten Reinheit, wobei der überwiegende Rest nur durch zu hohe Wassergehalte (> 1000 ppm) verunreinigt war.

### Beispiel 2

Beispiel 1 wurde wiederholt, allerdings war die Verunreinigung N-Methylsuccinimid (Verunreinigung V, R = H) mit 1000 ppm. Es resultierte das gleiche Ergebnis.

### Beispiel 3

300 mL des Einsatzstoffgemischs aus Beispiel 1 wurde in einem Autoklaven mit 1 Gew.-% Palladium (5 Gew.-%) auf Aktivkohle versetzt, 10 bar Wasserstoff aufgepresst, auf 100 °C für 5 h aufgeheizt. nach Abkühlen, Entspannen und Abfiltration des Katalysators wurde wie in Beispiel 1 aufgearbeitet. Es resultierten ca. 95 % an reinem NMP ohne die Verunreinigungen der Formel I und VI.

### Beispiel 4

Beispiel 3 wurde wiederholt, mit dem Unterschied, dass als Katalysator Nickel (10 Gew.-%) auf Aluminiumoxid verwendet wurde und die Hydrierung bei 130 °C durchgeführt wurde. Wiederum resultierten ca. 95 % reines NMP ohne die Verunreinigungen der Formel I und VI.

### Beispiel 5

Analog Beispiel 2 wurde N-Ethylpyrrolidon, das mit 500 ppm N-Ethylsuccinimid (Verunreinigung V, R = Me) verunreinigt war, eingesetzt. Die Zusatzmenge an NaBH₄ betrug 1000 ppm. Es wurden über 95 % reines N-Ethylpyrrolidon erhalten, das kein N-Ethylsuccinimid mehr enthielt.

## Patentansprüche

1. Verfahren zur Reinigung von N-Alkyl-substituierten Pyrrolidonen, die eine oder mehrere der Verunreinigungen der Formel I bis VII enthalten, 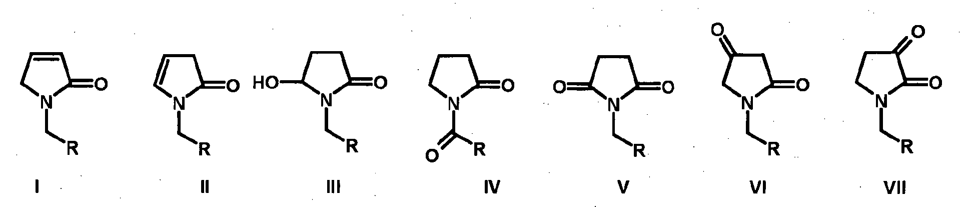 mit R ausgewählt aus der Gruppe Wasserstoff, linearen oder verzweigten C₁-C₂₀-Alkylgruppen,
umfassend folgende Schritte
I) Bereitstellung einer Mischung enthaltend mindestens ein N-Alkyl-substituiertes Pyrrolidon, mindestens eine Verbindung der Formel I bis VII in Mengen von 1 bis 20000 ppm
II) Hydrierung der Mischung aus Schritt I)
III) Destillation der erhaltenen Mischung aus Schritt II).

2. Verfahren nach Anspruch 1, wobei die Hydrierung in Schritt II) entweder durch Wasserstoff und einem Hydrierkatalysator oder durch ein komplexes Hydrid erfolgt.

3. Verfahren nach einem der Ansprüche 1 bis 2, wobei die komplexen Hydride ausgewählt sind aus der Gruppe von Natriumborhydrid und Lithiumaluminiumhydrid.

4. Verfahren nach einem der Ansprüche 1 bis 3, wobei das komplexe Hydrid in Mengen von 5 bis 50000 ppm bezogen auf die Menge an zu reinigender Mischung aus Schritt I) des erfindungsgemäßen Verfahrens zugesetzt wird.

5. Verfahren nach einem der Ansprüche 1 bis 4, wobei während einer Zeit von 0,1 bis 10 Stunden die Verunreinigung der Formel I bis VII während des Schritt II und/oder des Schritt III mit dem komplexen Hydrid bei gegebenenfalls wechselnden Temperaturen im Bereich von 50 bis 250 °C in Kontakt stehen.

6. Verfahren nach einem der Ansprüche 1 bis 2, wobei das Metall des Hydrierkatalysators ausgewählt ist aus der Gruppe von Cobalt, Rhodium, Ruthenium, Iridium, Nickel, Palladium, Platin, Kupfer, Silber, Gold und Rhenium.

7. Verfahren nach Anspruch 6, wobei der Druck bei der Hydrierung in Schritt II) im Bereich von Normaldruck bis 320 bar und die Temperatur im Bereich von 20 bis 250 °C liegt

8. Verfahren nach einem der Ansprüche 6 bis 7, wobei für den Schritt II) in Gegenwart eines heterogenen Hydrierkatalysators gearbeitet wird.

9. Verfahren nach einem der Ansprüche 1 bis 8, wobei die Destillation in Schritt III) kontinuierlich durchgeführt wird.

10. Verfahren nach einem der Ansprüche 1 bis 9, wobei die Destillation in Schritt III) in wenigstens zwei Kolonnen erfolgt.

11. Verfahren nach einem der Ansprüche 1 bis 10, wobei das N-Alkyl-substituierte-Pyrrolidon N-Methylpyrrolidon ist.

12. Verfahren nach Anspruch 9, bei dem die Destillation in Schritt III) bei Temperaturen im Bereich von 100 bis 250 °C und Drücken im Bereich von 300 bis 5000 mbar durchgeführt wird.

## Claims

1. A method for purifying N-alkyl-substituted pyrrolidones which comprise one or more of the contaminants of the formula I to VII, 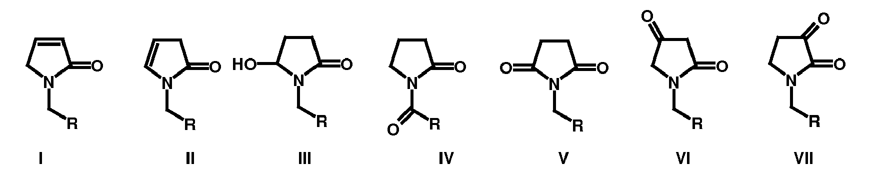 where R is selected from the group hydrogen, linear or branched C₁-C₂₀-alkyl groups,
comprising the following steps
I) provision of a mixture comprising at least one N-alkyl-substituted pyrrolidone, at least one compound of the formula I to VII in amounts of from 1 to 20 000 ppm
II) hydrogenation of the mixture from step I)
III) distillation of the mixture obtained from step II).

2. The method according to claim 1, where the hydrogenation in step II) takes place either by means of hydrogen and a hydrogenation catalyst or by means of a complex hydride.

3. The method according to either of claims 1 and 2, where the complex hydrides are selected from the group of sodium borohydride and lithium aluminum hydride.

4. The method according to any one of claims 1 to 3, where the complex hydride is added in amounts of from 5 to 50 000 ppm, based on the amount of mixture to be purified from step I) of the method according to the invention.

5. The method according to any one of claims 1 to 4, where the contaminants of the formula I to VII are in contact during step II and/or step III with the complex hydride at optionally varying temperatures in the range from 50 to 250°C over a period from 0.1 to 10 hours.

6. The method according to either of claims 1 and 2, where the metal of the hydrogenation catalyst is selected from the group of cobalt, rhodium, ruthenium, iridium, nickel, palladium, platinum, copper, silver, gold and rhenium.

7. The method according to claim 6, where the pressure during the hydrogenation in step II) is in the range from standard pressure up to 320 bar and the temperature is in the range from 20 to 250°C.

8. The method according to either of claims 6 and 7, where, for step II), processing is in the presence of a heterogeneous hydrogenation catalyst.

9. The method according to any one of claims 1 to 8, where the distillation in step III) is carried out continuously.

10. The method according to any one of claims 1 to 9, where the distillation in step III) takes place in at least two columns.

11. The method according to any one of claims 1 to 10, where the N-alkyl-substituted pyrrolidone is N-methylpyrrolidone.

12. The method according to claim 9, in which the distillation in step III) is carried out at temperatures in the range from 100 to 250°C and pressures in the range from 300 to 5000 mbar.

## Revendications

1. Procédé pour la purification de pyrrolidones substituées par alkyle sur l'atome N, qui contiennent un ou plusieurs des impuretés de formule I à VII, 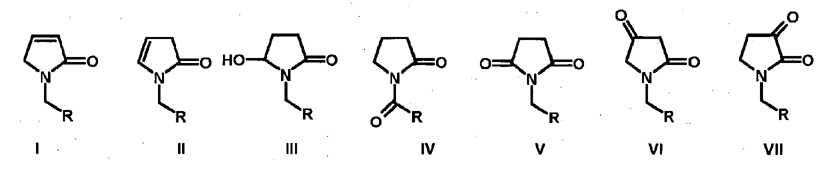 R étant choisi dans le groupe formé par l'hydrogène, les groupes C₁-C₂₀-alkyle linéaires ou ramifiés, comprenant les étapes suivantes
I) préparation d'un mélange contenant au moins une pyrrolidone substituée par alkyle sur l'atome N, au moins un composé de formule I à VII en des quantités de 1 à 20 000 ppm
II) hydrogénation du mélange de l'étape I)
III) distillation du mélange obtenu dans l'étape II).

2. Procédé selon la revendication 1, l'hydrogénation dans l'étape II) étant réalisée soit par de l'hydrogène et un catalyseur d'hydrogénation, soit par un hydrure complexe.

3. Procédé selon l'une quelconque des revendications 1 à 2, les hydrures complexes étant choisis dans le groupe formé par le borohydrure de sodium et l'hydrure de lithium-aluminium.

4. Procédé selon l'une quelconque des revendications 1 à 3, l'hydrure complexe étant ajouté en des quantités de 5 à 50 000 ppm par rapport à la quantité de mélange à purifier de l'étape I) du procédé selon l'invention.

5. Procédé selon l'une quelconque des revendications 1 à 4, les impuretés de formule I à VII étant en contact pendant un laps de temps de 0,1 à 10 heures pendant l'étape II et/ou l'étape III avec l'hydrure complexe, le cas échéant à des températures variables dans la plage de 50 à 250°C.

6. Procédé selon l'une quelconque des revendications 1 à 2, le mélange du catalyseur d'hydrogénation étant choisi dans le groupe formé par le cobalt, le rhodium, le ruthénium, l'iridium, le nickel, le palladium, le platine, le cuivre, l'argent, l'or et le rhénium.

7. Procédé selon la revendication 6, la pression lors de l'hydrogénation dans l'étape II) se situant dans la plage de la pression normale à 320 bars et la température se situant dans la plage de 20 à 250°C.

8. Procédé selon l'une quelconque des revendications 6 à 7, en travaillant, pour l'étape II), en présence d'un catalyseur d'hydrogénation hétérogène.

9. Procédé selon l'une quelconque des revendications 1 à 8, la distillation dans l'étape III) étant réalisée en continu.

10. Procédé selon l'une quelconque des revendications 1 à 9, la distillation dans l'étape III) ayant lieu dans au moins deux colonnes.

11. Procédé selon l'une quelconque des revendications 1 à 10, la pyrrolidone substituée par alkyle sur l'atome N étant la N-méthylpyrrolidone.

12. Procédé selon la revendication 9, la distillation dans l'étape III) étant réalisée à des températures dans la plage de 100 à 250°C et à des pressions dans la plage de 300 à 5000 mbars.
